# EUROPEAN PATENT SPECIFICATION

(11) **EP 1 817 072 B1**
(45) Date of publication and mention of the grant of the patent: **27.03.2013**
(21) Application number: 05791635.5
(22) Date of filing: 22.08.2005
(51) Int. Cl.: A61F 5/00, A61M 25/04

(54) **Apparatus for reducing obesity**
Gerät zur reduzierung von Adipositas
Appareil permettant de reduire l'obesite

(30) Priority: 30.11.2004 US 999410
(43) Date of publication of application: 15.08.2007
(73) Proprietor: BINMOELLER, Kenneth, Rancho Santa Fe, CA 92067 (US)
(72) Inventor: BINMOELLER, Kenneth, Rancho Santa Fe, CA 92067 (US)
(74) Representative: Dee, Ian Mark
(86) International application number: PCT/US2005/029932
(87) International publication number: WO 2006/060049

(56) References cited:
- WO-A-2004/041133
- WO-A-2005/120363
- DE-A1- 4 012 642
- JP-A- 1 015 063
- US-A- 5 820 584
- US-A1- 2003 040 804
- US-A1- 2004 122 456
- US-B2- 6 994 095

## Description

### BACKGROUND OF THE INVENTION

### Field of the Invention

The present invention relates to apparatus for curbing the appetite of persons being treated for obesity.

### DESCRIPTION OF THE RELATED ART

Extreme obesity is a major health concern in the United States and other countries. Its complications may include hypertension, diabetes, coronary artery disease, stroke, congestive heart failure, venous disease, multiple orthopedic problems and pulmonary insufficiency with markedly decreased life expectancy. Medical management including dietary, psychotherapy, medications and behavioral modification techniques have not yielded exceptional results in multiple trials. Despite the declaration of obesity as a major health problem, the Centers for Disease Control reports that obesity contributes to about 400,000 deaths annually, just behind tobacco (435,000) and ahead of alcohol (85,000), car accidents (43,000) and guns (29,000). Obesity and its complications now account for an estimated 9 percent of U.S. health spending.

Non-surgical approaches for the treatment of obesity include voluntary dieting which is often unsuccessful since most persons do not possess sufficient willpower to limit the intake of food. In addition to behavioral modification, several surgical techniques have been tried which induce malabsorption by reducing the absorptive surface of the small intestine or modify the stomach to reduce a patients desire to eat. Gastric reduction surgeries in which the stomach's volume is reduced had limited early success but often the stomach's size stretches over time so these patients did not exhibit real weight for a sustained period of time. Other surgical approaches combine gastric volume reduction by either partition or bypass with a reduction in the absorptive surface of the small intestine. These procedures may be both hazardous to perform in morbidly obese patients and often create numerous life-threatening postoperative complications. Such procedures typically are invasive, require a long recuperation time and subject the patient to undue pain and discomfort Also, such operative procedures are often difficult to reverse. These procedures are also expensive and place a large burden on the national health care system.

Other endoscopic approaches include implantation of gastric balloons that prevent overeating by occupying volume within the stomach. This fills a portion of the stomach and provides the patient with a feeling of fullness, thereby reducing food intake. Many problems are associated with the gastric balloon device, including poor patient tolerance and complications due to rupture, migration, and pressure trauma to the gastrointestinal tract. Some sham-controlled studies have failed to show that the gastric balloon was superior to diet alone in achieving weight reduction.

Other devices are designed to attempt to limit the absorption of nutrients in the duodenum by funneling the food through a tube so that the digestive process bypasses portions of the small intestine entirely. By interrupting the intermixing of the digestive fluids and/or limiting the residence period within the stomach, it is believed that the food materials will not fully digest into particles small enough to be absorbed by the body. However these devices have not been evaluated clinically.

WO 2004/041133 discloses an apparatus and method for treating morbid obesity. The apparatus comprises an elongate tubular body having a proximal end and a distal end, a funnel opening on the proximal end, and a support structure that is spaced distally apart from the proximal end.

US-5,820,584 discloses a duodenal insert comprising an elongated, open-ended tube and a pair of spaced-apart anchoring rings.

US 2003/0040804 discloses a device for inducing weight loss that comprises a tubular prosthesis that is capable of self-expansion from a collapsed configuration to an expanded configuration.

Having made the above critical observations, the present invention further recognizes a need for a transoral endoscopic device that mediates physiologic weight loss that is easily inserted into and removed from the gastrointestinal tract, well tolerated by the patient, does not migrate, does not adversely obstruct the lumen, and does not cause tissue injury.

### BRIEF SUMMARY OF THE INVENTION

The present invention provides a duoderal/small intestinal insert treatment of obesity according to claim 1. In one embodiment, the apparatus comprises a series of flow reduction elements in the small intestine to induce satiety. The flow reduction elements are attached along an elongated member which may or may not have a central lumen inside. This elongated member is used to position the flow reduction elements in the small intestine. The length and diameter of the flow reduction section can be selected by the physician to adjust the amount of weight reduction to the patients needs.

The central tube has an anchoring member attached near the proximal end that secures the proximal end in the antrum of the stomach. The anchoring member is sized so that it will not pass through the pyloric valve and so that it secures the central tube and the attached flow reduction elements in proper position in the small intestine. In one embodiment, the anchoring member is constructed of one or more inflatable balloons that when inflated are larger than the pylorus. The anchoring balloons can be deflated for delivery into the stomach and removed through the working lumen or alongside an endoscope. In another embodiment the anchoring member is an expandable umbrella-like skeleton frame that is attached to the flexible tube. The large end of the umbrella faces the pylorus and the frame can be collapsed for delivery and recovery.

The flow reduction elements can have various shapes and may be attached at various points along the central tube. The flow reduction elements may be inflated with fluid through a fluid connection with the central tube or may be constructed from self-expandable material such as a foam or spring structure. The space occupying flow reduction elements may also be filled or impregnated with pharmacologies, biochemicals, alimentary lipids, alimentary peptides or metabolic substances that release into the small intestine to further provide feelings of satiety.

The transoral gastric device can be inserted with a delivery catheter through the working lumen of an endoscope or alongside an endoscope and may be removed with the aid of an endoscope if desired.

### DETAILED DESCRIPTION OF THE INVENTION

The human stomach and small intestine include an esophagus, stomach, antrum, pylorus, pyloric valve, duodenum, jejunum and ampulla of Vater. The esophagus terminates at the nose or mouth at its superior end and at the stomach at its inferior end. The stomach encloses a chamber which is characterized, in part, by the esophageal-gastric juncture, which is an opening from the esophagus at one end, and the antrum-pyloric juncture which is a passageway between the antrum t through the pylorus to the duodenum at the other end. Specifically, the pylorus controls discharge from the stomach by a sphincter muscle, the pyloric valve, which enables the pylorus to open wide enough to pass an object which is approximately one cubic centimeter or less. Gastric contents, after passing into the duodenum, continue on into the jejunum and on into the ileum (not shown). The duodenum, jejunum and ileum make up what is known as the small intestine. However these individual portions of the alimentary canal are sometimes individually referred to as the small intestine. In the context of this invention the small intestine refers to all or part of the duodenum, jejunum and ileum. The ampulla of Vater is shown as a small protrusion on the medial wall. Bile and pancreatic fluids enter the duodenum at this point to further aid digestion.

The duodenum comprises the first 22.9 to 25.4 cms nine to ten inches of the small intestine and is the only portion of the small intestine which is attached to the back wall of the abdominal cavity (retroperitoneum). The remainder of the small intestine is not attached to the body, but merely folds freely in a sack called the mesentery, which is contained within the peritoneum. The digestive process starts when food materials are mixed with saliva and enzymes in the mouth. The digestive process continues in the stomach, where the food is combined with acids and additional enzymes to liquefy the food. This food resides in the stomach, for a short time and then it passes into the duodenum to be intermixed with bile and pancreatic juice, which make the nutrients contained therein available for absorption by the villi and microvilli of the small intestine or by other absorptive organs of the body.

The present invention understands that if the passage of partially digested food as described is partially blocked and the flow rate through the small intestine is reduced, then the emptying of the stomach and the duodenum will occur slower. This in turn will create a feeling of satiety and will decrease the consumption of food by an obese patient.

Additionally, because a large amount of the nutritional absorption occurs in the small intestine, if the amount of absorptive surface area of the walls of the small intestine is restricted or blocked, thus interrupting or reducing the intermixing of the digestive fluids, the partially digested food materials are not readily absorbed by the small intestine or other absorptive organs of the body. The partially digested food materials are then passed to the large intestine for elimination from the body with limited caloric absorption by the body.

Furthermore, the present invention understands that if the physical characteristics of the device and/or a reduction of flow rate of food breakdown products through the small intestine results in distension of the small intestine, or increases the contact time between the small intestine and the partially digested food then this distention or increased contact time may activate osmoreceptors that may release hormones and neurotransmitters such as cholecystokinins (CCK) and neural signals that may induce satiety.

In one embodiment, the small intestinal insert made in accordance with the present invention in has a proximal portion and a distal portion. The insert has a central tube that extends between the proximal portion and the distal portion. A series of flow reduction elements can be attached to the distal portion of the central tube and may be sized to fit inside the small intestine. However the area of the central tube near the ampulla of Vater has no flow reduction elements to prevent blockage of the ampulla of Vater. The central tube preferably has an anchoring member attached near a proximal end of the central tube, with the anchoring member securing the proximal end of the central tube in the antrum of the stomach. The anchoring member is sized so that it will not pass through the pylorus, so that it can maintain the flow reduction elements in proper position in the small intestine. In one embodiment, the anchoring member can be established by one or more inflatable balloons that when inflated are larger than the pylorus. The inflatable balloons can be deflated for delivery into the stomach and then inflated inside the stomach. The inflatable balloons can also be later deflated for removal using endoscopic techniques.

The central tube can be flexible and constructed of polymeric material that can be easily formed or extruded and delivered with the aid of an endoscope by known techniques. A central tube that is soft and flexible will contour to the anatomy of the gastrointestinal tract and provide less irritation of the stomach lining. The central tube can be made from polymers such as but not limited to nylon, polyolefins, polyurethane, silicone, polyvinyl chloride (PVC), Dacron®, latex, polyethylene, polypropylene, PVCD, polyethylene terephthalate (PET), teflon, and their mixtures and blocks or random copolymers. Further, the central tube may have a mono-layer, bi-layer or multi-layer construction. For instance, the central tube may have an inner layer of nylon or ethyl vinyl acetate and an outer layer of silicone for better biocompatibility. In addition, the substantially liquid-impermeable material could contain a radiopaque substance to enable visualization of the central tube in the patient's small intestine. Alternatively, the central tube could have band of radiopaque material like a metal foil around its circumference to enable visualization. The central tube polymer needs to be compatible with the chemical environment of the gastrointestinal tract and should provide enough structural integrity to prevent migration of the flow reduction elements in response to peristaltic action in the bowel.

The length of the central tube can be established depending on the therapeutic result desired and the patient's anatomy. For example, the central tube and the attached flow reduction elements may extend into a portion of or through the entire duodenum. On some patients the central tube and the attached flow reduction elements may extend past the duodenum and into the jejunum. It is anticipated that several lengths might be used by a physician to treat the various body types and metabolic demands. For example, if a patient is 20% overweight, the physician might select a length of central tube with its attached flow reduction elements that permits absorption of only 80% of the nutritional potential of a typical daily intake of calories. The reduction of caloric intake over time will lead to weight loss.

In one embodiment, the central tube has an outer wall and an inner wall that define an interior space. The interior space forms an inner lumen that may be continuous from the proximal end to just short of the distal end of the central tube. The distal end of the central tube is sealed at a point so that fluid introduced into the central tube does not leak out distally into the small intestine. In some embodiments a valve can be located substantially at the proximal end of the inner lumen. The valve may be a self sealing valve that has a septum that can be accessed by a needle or blunt tip tube for introduction of fluid into the inner lumen. The valve also can be accessed so that the fluid inside the inner lumen of the central tube can be aspirated for removal. It is to be understood that the valve type is not limited to a septum type valve only, and that other types of mechanical valves may also be used in place of the septum valve described.

One or more flow reduction elements can be attached to the central tube. In some embodiments the diameter of each flow reduction element can be concentric with the axis of the central tube. Each flow reduction element has an outer wall, an inner wall, and an inner space is established inside the inner wall. At or near its proximally-oriented surface and also at or near its distally-oriented surface, each flow reduction element can be attached to the central tube with the inner space of the flow reduction element in fluid communication with the lumen of the central tube, such that the inner space surrounds the outer wall of the central tube. Each flow reduction element may be attached to the central tube by adhesives, heat bonding, mechanical restraint or other suitable methods.

The central tube can be formed with plural inlet/exit ports that are located inside respective flow reduction elements. More specifically, each port is formed completely through the central tube wall to establish a pathway for fluid communication between the inner lumen of the central tube and the inner space of the respective flow reduction element. Consequently, the inner lumen of the central tube may be used to introduce fluid into the inner spaces of the flow reduction elements and to inflate the flow reduction elements from a collapsed configuration, in which insertion and removal of the flow reduction elements is facilitated, to an inflated configuration in which resistance to food passage is increased to induce satiety. Thus, the flow reduction element or elements in this embodiment act as balloons that can be deflated and collapsed around the central tube for introduction into the small intestine and then inflated to the desired diameter once in position.

Each flow reduction element or elements can either be elastic balloons or inelastic balloons. The balloon may be a elastic balloon formed of latex, polyurethane, polyethylene, polypropylene, PVC, PVCD, polyethylene terephthalate (PET), teflon, their mixtures and blocks or random copolymers may also be used, for example. The material may be non-elastic or semi-elastic, such as Dacron®, Nylon®, and the like. It may alternatively be a inelastic balloon formed of polyethylene. Further, the balloon may have a mono-layer, bi-layer or multi-layer construction. For instance, the balloon may have an inner layer of nylon or ethyl vinyl acetate and an outer layer of silicone for better biocompatibility. In addition, the substantially liquid-impermeable material could contain a radiopaque substance to enable visualization of the balloon in the patient's small intestine. Alternatively, the central tube could have band of radiopaque material like a metal foil around its circumference to enable visualization. When an elastic balloon material is used to establish a flow reduction element, the flow reduction element inflates to a diameter that is dependent on the volume of fluid introduced into the inner space of the flow reduction element. This embodiment permits adjustment of the balloon size as determined by the physician. If the balloon is too small, for instance, additional fluid could be introduced to enlarge the balloon diameter. Alternatively, if the balloon is too large, additional fluid could be removed to shrink the balloon diameter. It is understood that an alternate embodiment consisting of an inelastic balloon inflates to a diameter that is independent of the volume of fluid introduced into the inner space of the sphere. The diameter of this type of balloon is fixed when manufactured and does not permit in situ adjustment of the balloon size. However, this type of balloon prevents possible over inflation and rupture if too much fluid is introduced into the balloon.

The flow reduction elements can have the shape of a round sphere. However, other shapes are contemplated and any shape that effectively functions to inhibit the passage of partially digested food into the small intestine is acceptable. It is understood that the ability of the small intestinal insert to remain within the small intestine can be affected by the shape, orientation and tautness of the flow reduction elements. For example alternate shapes such as ovoid, elliptical, elongated ellipse and even irregular non-geometrical shapes are potentially feasible.

In an alternative embodiment of the present invention, one or more flow reduction elements are eccentrically attached to a central tube. In this embodiment the axis or diameter of the flow reduction element or elements is not concentric with the axis of the central tube. The outer wall of the flow reduction element is attached to the side of an outer wall of the central tube. An inner space of each flow reduction element is eccentric relative to the axis of the central tube and is in fluid communication with an inner lumen of the central tube through a respective opening.

In the embodiment, the inner lumen can be used to introduce and remove fluid into the inner space of the flow reduction element to move the flow reduction element between inflated and collapsed configurations.

In this context the flow reduction elements can be inflated with a fluid, either liquid or gas. Preferably the gas is air, nitrogen or carbon dioxide and the liquid is preferably water or water mixed with other solutions, such as sterile saline. It is important for the physician to monitor the flow reduction element location in the small intestine and the diameter of the flow reduction element relative to the diameter of the small intestine. The flow reduction element can be inflated with a radiopaque fluid that is visible on X-ray. If the flow reduction element containing the radiopaque fluid is visible on x-ray, the physician can non-invasively visualize the flow reduction element size from outside the patient's body. This knowledge enables the physician to adjust the size of the flow reduction element by injecting additional fluid into the flow reduction element through the inner lumen as required. Likewise radiopaque marker bands can be placed around the central tubes respectfully to facilitate visualization of the central tube's location in the small intestine. The radiopaque marker bands can be placed at predetermined intervals so that the distance inside the small intestine can be used as depth markers and can be measured from outside the body, and can be created by tantulum impregnated ink, or tantulum bands.

In an alternative embodiment there is provided a central shaft around which first flow reduction elements are concentrically attached and second flow reduction elements are eccentrically attached. The element can be attached to the central shaft in any manner as described previously. The flow reduction elements are made from material that can be folded or collapsed to a first volume suitable for insertion with the aid of an endoscope and then self expand to a second volume suitable for restricting the flow of partially digested food according to the present invention. The flow reduction elements can be made from materials such as sponge, foam, hydrogels or springs. The sponge can be composed of polyvinyl acetal polymer, neoprene, silicon, The foam can be composed of polyvinyl acetal polymer, The hydrogel can include any of the following: polysaccharides, proteins, polyphosphazenes, poly(oxyethylene)-poly(oxypropylene) block polymers, poly(oxyethylene)-poly(oxypropylene) block polymers of ethylene diamine, poly(acrylic acids), poly(methacrylic acids), copolymers of acrylic acid and methacrylic acid, poly(vinyl acetate), and sulfonated polymers. These materials can be compacted into a small volume and then self expand to a pre-determined shape and volume when unrestricted. The central shaft can be solid and without an inner lumen or inner space. Because the flow reduction elements self expand, the need for an inflation system is eliminated and this embodiment represents a simple mechanical design flow reduction elements are attached mechanically, by heat fusing, adhesives or other suitable methods as known in the art.

The surface of the flow reduction element and outside walls of the central tube may be associated with slow release medicaments, enzymes, cofactors, pharmacologics, biochemicals, alimentary lipids, alimentary peptides or metabolic substances. In this context, "associated with" means filled or coated, covalently bonded, hydrogen-bonded, layered, impregnated, incorporated into matrices, and other such methods of associating molecules, which will be well-known to one of skill in the art. These substances are designed to release over time into the intestine to modify the biochemical processes or trigger alternative receptor sites that in turn will alter the digestive process. Pharmacologics of interest include, but are not limited to, lipase-inhibitors such as orlistat (Xenical®); oradrenergic/serotonergic agents such as sibutramine (Meridia®, Reductil®); anti-depressants such as fluoxetine (Prozac®) and sertraline (Zoloft®), bupropion, (Amfebutamone®, Wellbutrin® and Zyban®); noradrenergic agents/amphetamines such as diethylpropion (Tenuate®, Tepanil®), diethylpropion ER (Tenuate Dospan®), and phemermine (Adipex-P®, Fastin®, Obestin-30®, Phentamine®, Zantryl®; Lonamin®, Oby-Cap®); anti-epileptic drugs such as Topiramate® and Zonisamide®, sympathomimetic amines such as phendimetrazine tatrate (Bontril®), benzphetamine (Didre®); and other drugs such as metformin (Glucophage®) and rimonabant, ephedrine, leptin, neuropeptide-Y receptor blockers.

It is important that the diameter of the central tube not obstruct the pyloric valve opening so that the valve can still function normally. The central tube diameter should be in the range of 1.67 to 2.3 mm 5 to 7 French). Distal to the pylorus and immediately after entering the duodenum the central tube can assume a sharp bend of radius β between the duodenal bulb and the vertical duodenum, and a sharp bend of radius α between the vertical duodenum and horizontal duodenum. Preferably the radius β and the radius α may be between about 45° and about 110°. More preferably, the radius β and the radius α may be between about 60° and about 100° such that the central tube bends to follow the inner lumen of the duodenum at this these locations. The central tubes pre-formed with a configuration that conforms to the duodenal angulations prior to insertion in the body and in one embodiment, is constrained in a straight configuration by a stiffening rod placed down the inner lumen of the central tube. This stiffening rod is placed into a separate lumen designed to house this stiffening rod or imbedded in the wall of the central tube. Upon insertion into the patient with the aid of an endoscope, when the central tube reaches the location of the sharp bends in the duodenum, the stiffening rod is withdrawn, thereby allowing the central tube to assume the pre-formed shape. In another embodiment, the central tube may have a shape memory alloy wire imbedded inside the central tube wall for residing in the inner lumen. This shape memory alloy wire has a pre-set bend configuration with a radius β and a radius α that matches the bend configuration of the duodenum and is positioned in the central tube at the corresponding location. Upon insertion into the patient with the aid of an endoscope, when the central tube reaches the location of the sharp bend in the duodenum and the shape memory alloy wire reaches a pre-set transition temperature equal to the temperature of the small intestine or 2.77°C (37° Fahrenheit), the wire assumes the programmed shape and forces the central tube and the central tube wall to assume the same shape. Shape memory alloy wire can be composed of metal alloys, including but not limited to, NiTi (Nickel - Titanium), CuZnAl, and CuAlNi. In another embodiment, the central tube may have a spring embedded inside the central tube wall or inner lumen. This spring could be pre-shaped to the anatomy of the wall of the small intestine. The spring is held straight during delivery and conforms to the small intestine anatomy after release. The shape enables the device to remain in place.

Turning to various anchoring members that can be used, the central tube has an anchoring member attached near the proximal end. The anchoring member can be established by one or more inflatable balloons. These balloons can be eccentrically attached to the central tube at point near the proximal end of the central tube. These balloons can be formed in many shapes and are not limited to the spherical shape shown. The central tube can be formed with an opening for each respective balloon so that a pathway for fluid communication is established between the inner lumen of the central tube and the inner space of each balloon. The inner lumen is used to introduce fluid into the inner space of the balloon and inflate the balloon from a first volume in a collapsed state to a second volume or inflated state.

When the anchoring member is fully inflated; it secures the proximal end of the central tube in the antrum of the stomach. The inflatable balloons have a combined cross sectional diameter greater than the diameter of the pyloric valve to prevent migration across the pylorus. The inflatable balloons can be inflated and deflated by adding or removing fluid from the central tube inner lumen. The inflatable balloons may be connected to the same central tube inner lumen as the flow reduction elements and can be inflated simultaneously and deflated simultaneously. However, the central tube may have more than one inner lumen so that the inflatable balloons and individual flow reduction elements may be inflated and deflated independently from each other.

In another embodiment the central tube is attached to an inverted umbrella skeleton for anchoring. This skeleton has a ring that surrounds the central tube and is supported by three struts. These struts are joined together at the central tube at a point and attached to the ring at points. It is possible to construct the anchoring member with one or more struts. The ring is made from flexible plastic material or flexible wire and has a diameter significantly larger than the diameter of the pyloric valve. The umbrella skeleton is collapsed around the central tube for insertion into the stomach with the aid of an endoscope. As the device is released from the endoscope, the umbrella skeleton springs out. The struts may be made from plastic, metal or from plastic covered metal. The edge of the ring which is in contact with the antrum walls, may be constructed to assist in securing the umbrella ring to the walls of the antrum. The surface of the ring may be roughened to increase surface friction or the wall may have protrusions or barbs that physically attach to the stomach lining.

In another embodiment of the current invention, the duodenal/small intestinal insert has a proximal portion and a distal portion. In this embodiment, a central shaft is attached to an expandable sleeve at the sleeve distal end near the distal portion of the duodenal/small intestinal insert. The opposite end of the central shaft is attached to a toroid anchoring member. The anchoring member is attached at the central shaft with two connecting struts. More than two connecting struts may be employed to securely attach the toroid anchoring member to the central shaft. The anchoring member is shaped like a funnel that is designed to seat in the Pylorus without obstructing its function. The central shaft is pre-formed to have a configuration that conforms to the anatomy of the duodenum. A central shaft so described would also force the expandable sleeve to assume the configuration of the shaft. The central shaft may be constructed out of wire, spring, shape memory alloys, hollow steel tubing or plastic polymers. The expandable sleeve is comprised of at least one flow reduction element and a connecting tube. The flow reduction element can be formed using springs or polymer materials. The flow reduction element may be formed from a spring and then covered with a flexible polymer to prevent partially digested food from entering the flow reduction element. The flow reduction element can be formed with a preset curved shape which can be straightened out for insertion with the aid of an endoscope. The flow reduction element diameter is sized to the small intestine diameter. When the connecting tube, anchoring member and the flow reduction element are in a collapsed configuration for insertion into the small intestine, the connecting tube and the expandable sleeve have been drawn toward the proximal end of the central shaft. The connecting tube also covers the collapsed anchoring member. This movement of the connecting tube relative to the central shaft occurs because the flow reduction element is collapsed in response to a force A applied to the connecting tube. It is anticipated that the connecting tube can be pulled toward the proximal portion of the central shaft to collapse the insert for insertion into the small intestine with the aid of an endoscope. Once in position, the force A is removed, the connecting tube returns toward the distal portion of the insert which releases the anchoring member from its constraint and allows the expandable sleeve to expand to its original diameter.

This invention has been described and specific examples of the invention have been portrayed. The use of those specifics is not intended to limit the invention in anyway.

## Claims

1. A duodenal/small Intestinal Insert for treating obesity in a human patient comprising:
an elongated member having a proximal end and a distal end, wherein the elongated member is preformed with a configuration that conforms to the angulations of the duodenum of the human patient and is capable of being straightened for introduction into the duodenum and then capable of returning to the preformed configuration;
an anchoring member engaged with said elongated member at the proximal end; and
at least one flow reduction element engaged with said elongated member.

2. A duodenal/small intestinal insert of claim 1, wherein a portion of the elongated member is rigid.

3. A duodenal/small intestinal insert of claim 1, wherein a portion of the elongated member is flexible.

4. A duodenal/small intestinal insert of claim 1, wherein the elongated member is formed from shape memory materials.

5. A duodenal/small intestinal insert of claim 1, wherein the elongated member contains shape memory materials.

6. A duodenal/small intestinal insert of claim 1, wherein the elongated member is formed from a spring material.

7. A duodenal/small intestinal Insert of claim 1, wherein the flow reduction element is concentric with the elongated member.

8. A duodenal/small intestinal insert of claim 1, wherein the flow reduction element is eccentric to the elongated member.

9. A duodenal/small intestinal insert of claim 1, 1, wherein the elongated member is a tube with an inner lumen.

10. A duodenal/small intestinal insert of claim 9, wherein the flow reduction element and/or anchoring member can be inflated from a first volume to a second volume with a fluid introduced into the inner lumen of the elongated member at the proximal end.

11. A duodonal/small intestinal insert of claim 10, wherein the fluid is a gas.

12. A duodenal/small intestinal insert of claim 1, wherein the flow reduction element comprises a spring.

13. A duodenal/small intestinal insert of claim 1, wherein the flow reduction element self-expands from a first volume to a second volume when unrestrained.

14. A duodenal/small intestinal insert of claim 1, wherein the anchoring member is configured to reside in the antrum of the human patient and when fully deployed restrict distal migration of the elongated member through the small intestine of the human patient.

15. A duodenal/small intestinal insert of claim 14, wherein the anchoring member comprises two or more balloons eccentrically arranged on the proximal end of the elongated member.

16. A duodenal/small intestinal insert of claim 15, wherein the balloons are inflatable and are capable of being inflated to a combined diameter is larger than the opening of the pylorus.

17. A duodenal/small intestinal insert of claim 14 wherein the anchoring member comprises:
collapsible, umbrella cage with a closed end and an open end, said closed end connected to the proximal end of the elongated member, the open end oriented toward the distal end of the elongated member, and the open end of the umbrella cage is attached to a toroid.

18. The anchoring member of claim 17, wherein the umbrella cage is comprised of a shape memory material.

19. The anchoring member of claim 18, wherein the umbrella cage is comprised of an alloy of NiTi, CuZnAl or CuAlNi.

20. The anchoring member of claim 17, wherein, wherein the umbrella cage is constructed from a spring material.

21. A duodenal/small intestinal according to claim 10, wherein a self sealing valve is positioned at a proximal opening of the elongated member, said valve adapted to control the amount of fluid introduced or released from the inner lumen of said elongated member and an opening at the distal end of the elongated member is sealed; and wherein the flow reduction member(s) and/or anchor member is capable of being expanded by the introduction of fluid into the inner lumen of the elongated member.

## Patentansprüche

1. Duodenaler/kleiner Darmeinsatz zur Behandlung von Fettleibigkeit bei einem menschlichen Patienten, der umfasst:
ein längliches Element, das ein proximales Ende und ein distales Ende aufweist, wobei das längliche Element mit einer Konfiguration vorgeformt ist, die mit den Angulationen des Zwölffingerdarms des menschlichen Patienten übereinstimmt, und in der Lage ist, zur Einführung in den Zwölffingerdarm ausgerichtet zu werden, und dann in der Lage ist, in die vorgeformte Konfiguration zurückzukehren;
ein Verankerungselement, das mit dem länglichen Element an dem proximalen Ende in Eingriff steht; und
mindestens ein Strömungsverringerungselement, das mit dem länglichen Element in Eingriff steht.

2. Duodenaler/kleiner Darmeinsatz nach Anspruch 1, bei dem ein Abschnitt des länglichen Elements starr ist.

3. Duodenaler/kleiner Darmeinsatz nach Anspruch 1, bei dem ein Abschnitt des länglichen Elements biegsam ist.

4. Duodenaler/kleiner Darmeinsatz nach Anspruch 1, bei dem das längliche Element aus Formgedächtnismaterialien gebildet ist.

5. Duodenaler/kleiner Darmeinsatz nach Anspruch 1, bei dem das längliche Element Formgedächtnismaterialien enthält.

6. Duodenaler/kleiner Darmeinsatz nach Anspruch 1, bei dem das längliche Element aus einem Federmaterial gebildet ist.

7. Duodenaler/kleiner Darmeinsatz nach Anspruch 1, bei dem das Strömungsverringerungselement mit dem länglichen Element konzentrisch ist.

8. Duodenaler/kleiner Darmeinsatz nach Anspruch 1, bei dem das Strömungsverringerungselement zu dem länglichen Element exzentrisch ist.

9. Duodenaler/kleiner Darmeinsatz nach Anspruch 1, bei dem das längliche Element ein Schlauch mit einem inneren Lumen ist.

10. Duodenaler/kleiner Darmeinsatz nach Anspruch 9, bei dem das Strömungsverringerungselement und/oder das Verankerungselement von einem ersten Volumen zu einem zweiten Volumen mit einem Fluid aufgeblasen werden kann, das in das innere Lumen des länglichen Elements an dem proximalen Ende eingeführt wird.

11. Duodenaler/kleiner Darmeinsatz nach Anspruch 10, bei dem das Fluid ein Gas ist.

12. Duodenaler/kleiner Darmeinsatz nach Anspruch 1, bei dem das Strömungsverringerungselement eine Feder umfasst.

13. Duodenaler/kleiner Darmeinsatz nach Anspruch 1, bei dem sich das Strömungsverringerungselement von einem ersten Volumen zu einem zweiten Volumen selbst expandieren kann, wenn es nicht zurückgehalten wird.

14. Duodenaler/kleiner Darmeinsatz nach Anspruch 1, bei dem das Verankerungselement konfiguriert ist, um in dem Antrum des menschlichen Patienten zu liegen, und, wenn es vollständig eingesetzt ist, die distale Migration des länglichen Elements durch den Dünndarm des menschlichen Patienten einschränkt.

15. Duodenaler/kleiner Darmeinsatz nach Anspruch 14, bei dem das Verankerungselement zwei oder mehr Ballone umfasst, die auf dem proximalen Ende des länglichen Elements exzentrisch angeordnet sind.

16. Duodenaler/kleiner Darmeinsatz nach Anspruch 15, bei dem die Ballone aufblasbar und in der Lage sind, auf einen kombinierten Durchmesser aufgeblasen zu werden, der größer als die Öffnung des Pylorus ist.

17. Duodenaler/kleiner Darmeinsatz nach Anspruch 14, bei dem das Verankerungselement umfasst:
einen kollabierbaren Schirmkäfig mit einem geschlossenen Ende und einem offenen Ende, wobei das geschlossene Ende mit dem proximalen Ende des länglichen Elements verbunden ist, das offene Ende zu dem distalen Ende des länglichen Elements hin orientiert ist und das offene Ende des Schirmkäfigs an einem Toroid befestigt ist.

18. Verankerungselement nach Anspruch 17, bei dem der Schirmkäfig aus einem Formgedächtnismaterial besteht.

19. Verankerungselement nach Anspruch 18, bei dem der Schirmkäfig aus einer Legierung von NiTi, CuZnAl oder CuAlNi besteht.

20. Verankerungselement nach Anspruch 17, bei dem der Schirmkäfig aus einem Federmaterial aufgebaut ist.

21. Duodenaler/kleiner Darmeinsatz gemäß Anspruch 10, bei dem ein selbstabdichtendes Ventil an einer proximalen Öffnung des länglichen Elements positioniert ist, wobei das Ventil angepasst ist, um die Menge an Fluid zu steuern, die aus dem inneren Lumen des länglichen Elements eingeführt oder freigesetzt wird, und eine Öffnung an dem distalen Ende des länglichen Elements abgedichtet ist; und wobei das/die Strömungsverringerungselement(e) und/oder das Verankerungselement in der Lage ist/sind, durch die Einführung von Fluid in das innere Lumen des länglichen Elements expandiert zu werden.

## Revendications

1. Insert pour le duodénum/petit intestin afin de traiter l'obésité chez un patient humain comprenant :
un élément allongé ayant une extrémité proximale et une extrémité distale, l'élément allongé étant préformé selon configuration qui se conforme aux angulations du duodénum du patient humain et étant adapté pour se redresser pour l'introduction dans le duodénum et pour revenir ensuite à la configuration préformée ;
un élément d'ancrage en prise avec ledit élément allongé au niveau de l'extrémité proximale ; et
au moins un élément de réduction d'écoulement en prise avec ledit élément allongé.

2. Insert pour le duodénum/petit intestin selon la revendication 1, dans lequel une partie de l'élément allongé est rigide.

3. Insert pour le duodénum/petit intestin selon la revendication 1, dans lequel une partie de l'élément allongé est flexible.

4. Insert pour le duodénum/petit intestin selon la revendication 1, dans lequel l'élément allongé est formé à partir de matériaux à mémoire de forme.

5. Insert pour le duodénum/petit intestin selon la revendication 1, dans lequel l'élément allongé contient des matériaux à mémoire de forme.

6. Insert pour le duodénum/petit intestin selon la revendication 1, dans lequel l'élément allongé est formé à partir d'un matériau à ressort.

7. Insert pour le duodénum/petit intestin selon la revendication 1, dans lequel l'élément de réduction d'écoulement est concentrique par rapport à l'élément allongé.

8. Insert pour le duodénum/petit intestin selon la revendication 1, dans lequel l'élément de réduction d'écoulement est excentrique par rapport à l'élément allongé.

9. Insert pour le duodénum/petit intestin selon la revendication 1, dans lequel l'élément allongé est un tube avec une lumière interne.

10. Insert pour le duodénum/petit intestin selon la revendication 9, dans lequel l'élément de réduction d'écoulement et/ou l'élément d'ancrage peuvent être gonflés d'un premier volume à un second volume avec un fluide introduit dans la lumière interne de l'élément allongé au niveau de l'extrémité proximale.

11. Insert pour le duodénum/petit intestin selon la revendication 10, dans lequel le fluide est un gaz.

12. Insert pour le duodénum/petit intestin selon la revendication 1, dans lequel l'élément de réduction d'écoulement comprend un ressort.

13. Insert pour le duodénum/petit intestin selon la revendication 1, dans lequel l'élément de réduction d'écoulement s'auto-expanse d'un premier volume à un second volume lorsqu'il est non contraint.

14. Insert pour le duodénum/petit intestin selon la revendication 1, dans lequel l'élément d'ancrage est configuré pour se trouver dans l'antre du patient humain et, lorsqu'il est complètement déployé, limiter la migration distale de l'élément allongé à travers le petit intestin du patient humain.

15. Insert pour le duodénum/petit intestin selon la revendication 14, dans lequel l'élément d'ancrage comprend deux ballonnets ou plus agencés de manière excentrique sur l'extrémité proximale de l'élément allongé.

16. Insert pour le duodénum/petit intestin selon la revendication 15, dans lequel les ballonnets sont gonflables et peuvent être gonflés à un diamètre combiné qui est supérieur à l'ouverture du pylore.

17. Insert pour le duodénum/petit intestin selon la revendication 14, dans lequel l'élément d'ancrage comprend : une cage repliable en forme de parapluie avec une extrémité fermée et une extrémité ouverte, ladite extrémité fermée étant raccordée à l'extrémité proximale de l'élément allongé, l'extrémité ouverte étant orientée vers l'extrémité distale de l'élément allongé, et l'extrémité ouverte de la cage en forme de parapluie est fixée à un tore.

18. Elément d'ancrage selon la revendication 17, dans lequel la cage en forme de parapluie est composée d'un matériau à mémoire de forme.

19. Elément d'ancrage selon la revendication 18, dans lequel la cage en forme de parapluie est composée d'un alliage de NiTi, CuZnAl ou CuAlNi.

20. Elément d'ancrage selon la revendication 17, dans lequel la cage en forme de parapluie est construite à partir d'un matériau à ressort.

21. Insert pour le duodénum/petit intestin selon la revendication 10, dans lequel la valve autoclave est positionnée au niveau de l'ouverture proximale de l'élément allongé, ladite valve étant adaptée pour contrôler la quantité de fluide introduit ou libéré de la lumière interne dudit élément allongé et une ouverture de l'extrémité distale de l'élément allongé est hermétiquement fermée ; et dans lequel l'élément (les éléments) de réduction d'écoulement et/ou l'élément d'ancrage peuvent être expansés par l'introduction du fluide dans la lumière interne de l'élément allongé.
